# DEMANDE DE BREVET EUROPEEN

(11) **EP 1 310 212 A1**
(43) Date de publication de la demande: **14.05.2003**
(21) Numéro de dépôt: 02292753.7
(22) Date de dépôt: 05.11.2002
(51) Int. Cl.: A61B 5/103, G01N 13/00

(54) **Procédé et dispositif pour l'évaluation de la sécheresse cutanée notamment**

(30) Priorité: 07.11.2001 FR 0114393
(71) Demandeur: L'OREAL, 75008 Paris (FR)
(72) Inventeur: Pruche, Francis, 60300 Senlis (FR); Rubinstenn, Gilles, 75005 Paris (FR)
(74) Mandataire: Tanty, François

(57) **Abrégé**

La présente invention concerne un procédé permettant de déterminer un type de peau, comportant les étapes suivantes :
- appliquer sur une zone de peau ou sur un organe de collecte préalablement mis au contact de ladite zone au moins une goutte (G) d'un produit apte à modifier au moins une propriété physico-chimique de la surface de ladite zone ou de l'organe de collecte exposée audit produit,
- évaluer, après disparition ou enlèvement de la goutte, l'étendue de ladite surface,
- déterminer en fonction de ladite étendue le type de peau.

## Description

La présente invention concerne, notamment, la détermination des types de peau.

L'expérience montre que les consommateurs ne choisissent pas toujours de manière optimale les produits de soins de la peau, parce qu'ils ne connaissent pas précisément leur type de peau et ses besoins spécifiques.

Il existe par conséquent un besoin pour permettre la détermination du type d'une peau, par exemple savoir déterminer si la peau est sèche ou grasse, afin notamment d'évaluer ses besoins en lipides et/ou en eau, la meilleure connaissance des besoins en lipides et/ou en eau pouvant guider le formulateur dans la préparation de produits ciblés, voire personnalisés.

On a proposé dans les brevets US 5 094 248 et 5 433 214 des dispositifs à appliquer sur la peau pour collecter le sébum.

On a proposé par ailleurs dans l'article "Sebum and stratum corneum lipids increase human skin surface free energy as determined from contact angle measurement: A study on two anatomical sites", dans la revue Colloïd and Surface B: Bio interfaces 8 (1997), 147-155, de mesurer l'angle de contact d'une goutte déposée sur la peau avec la surface de cette dernière, afin de mettre en évidence des différences entre la peau du front et celle de l'avant-bras.

L'invention vise à accroître encore la diversité des procédés et dispositifs à la disposition du public et des professionnels pour déterminer les types de peau et à les rendre suffisamment simples pour permettre une utilisation à grande échelle.

L'invention a notamment pour objet, selon un de ses aspects parmi d'autres, un procédé permettant de déterminer un type de peau, comportant les étapes suivantes :
- appliquer sur une zone de peau ou sur un organe de collecte de sébum préalablement mis au contact de ladite zone au moins une goutte d'un produit apte à modifier au moins une propriété physico-chimique de la surface de ladite zone ou de l'organe de collecte exposée audit produit,
- évaluer après disparition ou enlèvement de la goutte, par exemple par observation des modifications d'aspect induites par ledit produit, l'étendue de ladite surface,
- déterminer en fonction de ladite étendue le type de peau.

Un tel procédé présente l'avantage de pouvoir être facilement mis en oeuvre tout en permettant d'obtenir une information suffisamment précise pour permettre un diagnostic aisé.

Selon un aspect de l'invention, la goutte de produit est appliquée directement sur la zone de peau dont on souhaite déterminer le type.

Selon un autre aspect de l'invention, la goutte de produit est appliquée sur un organe de collecte préalablement mis en contact avec la zone de peau susceptible de transférer du sébum sur l'organe de collecte. Cet organe de collecte peut être une partie du corps humain ou non.

L'organe de collecte peut être constitué par exemple par une région du bras, par exemple l'avant-bras. Ainsi, dans un exemple de mise en oeuvre de l'invention, la personne dont on cherche à déterminer le type de peau peut passer par exemple son avant-bras sur son front, de sorte que le sébum présent à la surface du front est transféré sur l'avant-bras, puis une goutte de produit est déposée sur l'avant-bras, là où le sébum a été transféré. Une telle démarche est avantageuse notamment lorsque l'on cherche à éviter de déposer une goutte de produit sur le visage, par exemple. De plus, des évaluations comparatives peuvent être effectuées, en comparant par exemple les résultats d'une évaluation sur l'organe de collecte avant transfert du sébum et d'une évaluation sur l'organe de collecte après transfert. On peut encore comparer, par exemple, les résultats d'une évaluation sur un avant-bras après passage sur le front et d'une évaluation sur l'autre avant-bras.

L'organe de collecte peut également être constitué par un support indépendant du corps humain, par exemple une feuille, notamment une feuille comportant des fibres cellulosiques. Une telle feuille peut comporter des graduations ou une échelle préimprimée, par exemple.

Selon un aspect de l'invention, le produit utilisé est un produit coloré ou apte à produire une coloration sur la peau.

Lorsque le produit utilisé est un produit coloré, la coloration du produit est par exemple obtenue par incorporation dans un solvant, notamment un solvant aqueux, de l'un des colorants suivants : Erythrosine B, indigo carmine, permanganate de potassium, tanins, notamment les produits de l'oxydation des polyphénols, mélanines, henné, anthocyanes, produits fluorescents, notamment le chlorure de Dansyl.

Le produit utilisé peut également présenter des propriétés magnétiques. A cet effet, le produit utilisé peut comporter par exemple des particules magnétiques, notamment des particules contenant du fer, éventuellement associées à un polymère, un copolymère ou une matière plastique, tels que par exemple de la cellulose, du polybutène ou du polystyrène.

Le produit utilisé peut encore comporter au moins un anticorps, notamment un anticorps spécifique des kératines. Cet anticorps peut être associé à l'un des éléments de la liste suivante : particules magnétiques, composés fluorescents, enzymes (par exemple une péroxydase), système de type "ELISA".

Le produit utilisé peut encore être apte à modifier le microrelief de la peau.

Le produit utilisé peut comporter comme solvant de l'eau, un alcool, une huile, entre autres.

Le volume d'une goutte de produit déposée sur la peau peut être compris par exemple entre 5 et 100 µl, voire entre 10 et 60 µl, et être par exemple égal à environ 20 µl.

Selon un aspect de l'invention, la goutte est enlevée au moyen d'un élément absorbant, tel qu'une bandelette de papier buvard ou un coton tige, par exemple. D'autres moyens peuvent être utilisés sans que l'on sorte du cadre de la présente invention, par exemple un enlèvement de la goutte au moyen d'une aspiration. On peut également laisser la goutte disparaître par évaporation, par exemple. Il peut s'avérer toutefois préférable de procéder à l'enlèvement de la goutte plutôt que d'attendre que celle-ci ne s'évapore.

L'étendue de la surface de la peau ou de l'organe de collecte dont les propriétés ont été modifiées par la goutte de produit peut être mesurée de multiples manières après disparition ou enlèvement de la goutte, selon la nature du changement intervenu.

Cette étendue peut être évaluée in situ, en mesurant directement sur la peau ou sur l'organe de collecte, par exemple, ou en variante être évaluée après transfert sur un support d'une trace laissée sur la peau ou l'organe de collecte par ladite goutte de produit.

Dans le cas par exemple de l'utilisation d'un produit coloré ou apte à produire une coloration sur la peau ou l'organe de collecte, la taille de la tache qui a été laissée par la goutte à la surface de la peau ou de l'organe de collecte peut être mesurée au moyen par exemple d'un compas, d'une caméra, d'un rayon lumineux ou d'une échelle, graduée ou non, éventuellement associée à une optique, cette liste de moyens pouvant être utilisés n'étant pas limitative.

Dans le cas où le contact de la goutte de produit avec la peau ou avec l'organe de collecte laisse une trace mesurable non optiquement, par exemple parce que le microrelief ou les propriétés électriques ou magnétiques de la peau ou de l'organe de collecte ont été modifiées, on peut utiliser par exemple au moins un capteur magnétique ou au moins un capteur non optique apte à délivrer une image du microrelief de la peau ou de l'organe de collecte.

Le cas échéant, le résultat de la mesure peut être transmis à un ordinateur à des fins d'analyse. Le résultat de la mesure peut être entré dans l'ordinateur par le biais du clavier ou d'un écran tactile, par exemple. Le résultat de la mesure peut encore être transmis directement à l'ordinateur par l'appareil ayant servi à effectuer la mesure, par une liaison filaire ou par une liaison sans fil.

Le résultat de la mesure peut, le cas échéant, être transmis à distance par un réseau informatique, notamment le réseau Internet, à un centre de diagnostic, par exemple.

La goutte de produit peut être appliquée par exemple sur le front, le haut du torse, l'avant-bras, le dessus de l'épaule, entre autres. On peut effectuer des tests sur différentes parties du corps et tirer du recoupement des résultats des différents tests une information utile pour déterminer le type de peau.

On peut également appliquer successivement sur la peau ou l'organe de collecte des produits différents, présentant des affinités diverses à l'égard des lipides et/ou de l'eau, et tirer du recoupement des résultats des différents tests une information utile pour déterminer le type de peau. On peut ainsi, par exemple, utiliser successivement un produit aqueux puis un produit huileux, ou inversement.

Selon un aspect de l'invention, un soin est préconisé.

L'invention a encore pour objet, selon un autre de ses aspects, un procédé de traitement, par exemple cosmétique, de la peau, comportant les étapes suivantes :
- appliquer sur une zone de peau ou sur un organe de collecte préalablement mis au contact de ladite zone au moins une goutte d'un produit de test apte à modifier au moins une propriété physico-chimique de la surface de ladite zone ou de l'organe de collecte exposée audit produit, notamment un produit coloré ou apte à produire une coloration sur la peau,
- évaluer, après disparition ou enlèvement de la goutte, l'étendue de ladite surface,
- déterminer en fonction de ladite étendue le type de peau,
- traiter la peau avec un produit de traitement, notamment cosmétique, en fonction du type de peau ainsi déterminé.

Pour comprendre ce que l'on entend par cosmétique au sens de la présente demande, on pourra se référer à la Directive cosmétique 76/768/CEE.

Par « traitement cosmétique » on entend tout traitement non thérapeutique au moyen d'un produit cosmétique tel que défini dans la Directive ci-dessus.

Le traitement peut par exemple avoir pour conséquence de modifier l'affinité de la peau avec le produit de test, de sorte qu'une nouvelle opération de détermination du type de peau, après application du soin, peut conduire à un résultat différent.

On peut choisir la région du corps ou du visage où le test est effectué en fonction de la nature du traitement qui sera éventuellement effectué suite à ce test.

Ainsi, la goutte de produit de test peut être appliquée par exemple sur l'avant-bras dans le cas où le produit de traitement est destiné à l'ensemble du corps, et la goutte de produit de test peut être appliquée par exemple sur le front dans le cas où le produit de traitement est destiné plus particulièrement au visage.

L'invention a encore pour objet un procédé pour déterminer l'efficacité d'un traitement, notamment un traitement apte à agir sur l'hydratation et/ou la teneur en lipides de la peau, comportant les étapes suivantes :
- appliquer sur une zone de peau ou sur un organe de collecte préalablement mis au contact de ladite zone au moins une goutte d'un produit apte à modifier au moins une propriété physico-chimique de la surface de ladite zone ou de l'organe de collecte au contact de ladite goutte,
- évaluer l'étendue de la surface de ladite zone ou de l'organe de collecte ayant été au contact de ladite goutte, après enlèvement ou disparition de celle-ci, par observation des modifications induites par ledit produit,
- effectuer le traitement,
- renouveler les étapes a) et b) sur la peau traitée,
- déduire de la comparaison des résultats avant et après traitement une indication relative à l'efficacité du traitement.

Selon un aspect de l'invention, dans le cas où l'étendue de la surface de la peau ou de l'organe de collecte exposée au produit correspond à une peau plus sèche qu'une peau considérée comme normale, on applique un produit apte à diminuer la sécheresse de la peau, et dans le cas où l'étendue de ladite surface correspond à une peau plus grasse qu'une peau considérée comme normale, on applique un produit apte à abaisser la teneur en sébum de la peau.

Si l'on souhaite par exemple évaluer l'influence sur la peau d'un produit de soins appliqué sur celle-ci, on peut mesurer, après l'enlèvement ou la disparition d'une goutte nouvellement appliquée sur la peau, l'étendue de la surface exposée au produit de test, et la comparer avec celle de la surface exposée au produit de test de la goutte initialement appliquée. On peut ainsi déterminer, par exemple, à partir de la nouvelle mesure si le produit de soins est adéquat.

L'invention a encore pour objet, selon un autre de ses aspects, un procédé pour évaluer l'âge physiologique de la peau, le film lipidique de la peau tendant à disparaître avec l'âge, caractérisé par le fait qu'il comporte les étapes suivantes :
- appliquer sur une zone de peau ou sur un organe de collecte préalablement mis au contact de ladite zone de peau au moins une goutte d'un produit apte à modifier au moins une propriété physico-chimique de la surface de la peau ou de l'organe de collecte exposée audit produit,
- évaluer, après disparition ou enlèvement de la goutte, par observation des modifications induites par ledit produit, l'étendue de la surface de peau ou de l'organe de collecte ayant été au contact de ladite goutte,
- déterminer en fonction de ladite étendue l'âge de la peau.

L'invention a encore pour objet un ensemble comportant un applicateur apte à appliquer sur la peau ou sur un organe de collecte une goutte d'un produit apte à modifier au moins une propriété physico-chimique de la peau ou de l'organe de collecte, notamment un produit coloré ou apte à produire une coloration sur la peau ou l'organe de collecte, et au moins une échelle ou un appareil de mesure pour évaluer, après disparition ou enlèvement de ladite goutte, l'étendue de la surface de la peau ou de l'organe de collecte exposée audit produit.

L'échelle peut être graduée ou non, et comporter par exemple un marquage apte à indiquer directement le type de peau.

Dans le cas d'un produit coloré ou apte à laisser une coloration sur la peau, l'échelle peut être associée à une optique, par exemple, permettant de visualiser simultanément une image agrandie de la tache et l'échelle.

L'échelle peut encore être portée par l'applicateur.

L'échelle peut également être autocollante.

L'échelle peut encore être présente sur un support sur lequel est destinée à être transférée une trace laissée à la surface de la peau ou de l'organe de collecte par la goutte de produit, après enlèvement ou disparition de celle-ci. L'échelle peut alors être par exemple imprimée sur un tel support.

Le support en question peut par exemple se présenter sous la forme d'une carte réponse à adresser par courrier à un centre de diagnostic. Le support peut encore constituer une bandelette à introduire dans un appareil de lecture, par exemple.

Le support peut présenter une face adhésive, destinée à faciliter le transfert sur le support de la trace laissée par la goutte de produit à la surface de la peau ou de l'organe de collecte.

Le support peut encore être imprégné d'un révélateur apte à réagir au contact du produit constituant la goutte, de sorte que lors du transfert sur le support de la trace de produit laissée sur la peau ou l'organe de collecte, il se produise une réaction sur le support, par exemple une réaction colorée.

Eventuellement, le support peut permettre de collecter, lorsqu'il présente une surface adhésive, des particules de peau morte ou d'autres impuretés présentes à la surface de la peau, et le centre de diagnostic peut délivrer une information concernant le type de peau en recoupant l'information liée à l'étendue de la trace transférée sur le support et l'information liée à la quantité d'impuretés déposées sur le support, dans la zone de la surface adhésive située autour de la trace transférée.

Le support peut comporter, le cas échéant, une feuille de protection apte à recouvrir la surface adhésive après transfert de la trace, une telle feuille de protection présentant par exemple une surface traitée pour ne pas adhérer fortement à la surface adhésive.

L'image d'une tache formée sur le support par transfert peut encore être transmise à distance, par télécopie ou au moyen d'une caméra ou d'un scanner, à un centre de diagnostic.

L'applicateur peut comporter un réservoir de produit et une partie sécable ou destinée à être coupée ou percée.

Le réservoir de produit peut être formé au contact d'au moins une paroi souple. Le réservoir de produit peut être formé par exemple entre deux feuilles superposées.

L'applicateur peut comporter un conduit par lequel sort la goutte, de diamètre intérieur compris entre 50 µm et 3 mm, par exemple, et compris entre environ 0,1 mm et environ 2 mm, de préférence.

Lorsqu'un appareil de mesure de l'étendue de la surface de la peau dont les propriétés ont été modifiées par la goutte de produit de test est utilisé, cet appareil peut par exemple émettre un rayon lumineux en direction de la peau et capter la lumière réfléchie. Le rayon lumineux peut être produit par exemple par au moins une diode laser ou au moins une diode électroluminescente. Un tel rayon lumineux peut permettre par exemple de détecter une modification des propriétés réflexives ou colorimétriques de la surface de peau ou de l'organe de collecte exposée à la goutte de produit.

L'appareil de mesure peut aussi comporter, par exemple, au moins une barrette de capteurs associée à une optique permettant de projeter sur la barrette une image d'une tache laissée sur la peau ou de l'organe de collecte par la goutte de produit.

L'appareil de mesure peut comporter, le cas échéant, des moyens d'éclairage permettant d'éclairer la peau avec une lumière de composition spectrale choisie de manière à exciter la fluorescence d'un traceur. L'appareil de mesure peut ainsi comporter, par exemple, des moyens d'éclairage délivrant une lumière infrarouge ou ultraviolette.

L'appareil de mesure peut également comporter une caméra, par exemple une caméra reliée à un ordinateur personnel.

L'appareil de mesure peut aussi comporter au moins un capteur magnétique.

L'appareil de mesure peut également comporter au moins un capteur non optique, apte à délivrer une image des microreliefs de la peau ou de l'organe de collecte.

L'appareil de mesure peut encore, par exemple, comporter une interface permettant de transmettre des données à un ordinateur personnel et/ou à un réseau informatique.

L'invention a encore pour objet un appareil de mesure d'une étendue de la surface de la peau ou de l'organe de collecte exposée à une goutte d'un produit apte à modifier au moins une caractéristique physico-chimique de la peau ou de l'organe de collecte, notamment un produit coloré, ou apte à produire une coloration sur la peau ou l'organe de collecte, cet appareil comportant des moyens de mesure de l'étendue de ladite surface et des moyens d'affichage d'un type de peau en fonction du résultat de la mesure. Cet appareil peut par exemple délivrer une information non numérique du type "peau sèche", "peau normale" ou "peau grasse" ou en variante une valeur numérique, par exemple un score compris entre *n* à *m*, où *n* et *m* sont des entiers, le chiffre *n* correspondant à une peau très sèche et le chiffre *m* à une peau très grasse.

L'invention a encore pour objet une échelle comportant un marquage propre à indiquer directement le type de peau.

L'invention a encore pour objet un support destiné au transfert d'une trace laissée sur la peau ou l'organe de collecte par une goutte de produit et comportant un marquage propre à indiquer directement le type de peau.

L'invention a encore pour objet un compas comportant des indications propres à indiquer directement le type de peau.

L'invention a également pour objet un kit comportant un ensemble comportant un applicateur et une échelle ou un appareil de mesure, tel que défini plus haut, et un produit de traitement à appliquer sur la peau, notamment un produit de soins.

L'invention pourra être mieux comprise à la lecture de la description détaillée qui va suivre, d'exemples de mise en oeuvre non limitatifs, et à l'examen du dessin annexé, qui fait partie intégrante de la description, et sur lequel :
- la figure 1 représente de manière schématique un applicateur pouvant être utilisé pour appliquer une goutte de produit sur la peau,
- la figure 2 illustre l'application d'une goutte de produit sur la peau au moyen de l'applicateur de la figure 1,
- la figure 3 illustre l'enlèvement de la goutte de produit au moyen d'une bandelette de papier absorbant,
- la figure 4 représente de manière schématique une tache laissée à la surface de la peau, après enlèvement de la goutte,
- les figures 5 et 6 sont des représentations schématiques illustrant la différence de surface de contact entre la goutte et la peau selon le caractère plus ou moins gras de celle-ci,
- la figure 7 représente un autre exemple de dispositif permettant d'appliquer une goutte sur la peau et de mesurer la taille de la tache résultante,
- la figure 8 représente de manière schématique un dispositif de mesure de la dimension d'une tache laissée sur la peau par la goutte de produit,
- la figure 9 illustre la collecte d'informations et la transmission à un serveur distant,
- les figures 10 à 12 sont différents schémas en blocs illustrant des exemples de mise en oeuvre de l'invention,
- la figure 13 représente un kit comportant d'une part un dispositif permettant d'appliquer une goutte de produit sur la peau et de mesurer la taille d'une tache laissée sur la peau, et d'autre part un produit de soins,
- la figure 14 représente de manière schématique un compas pour mesurer la taille d'une tache,
- la figure 15 représente de manière schématique une échelle associée à une optique,
- la figure 16 représente, de manière schématique, une échelle comportant des indications renseignant directement sur le type de peau, et illustre son utilisation,
- la figure 17 représente de manière schématique un support pouvant servir à transférer une trace laissée sur la peau par une goutte de produit,
- la figure 18 représente de manière schématique un capteur non optique,
- la figure 19 représente un exemple d'organe de collecte, et
- la figure 20 représente un autre exemple d'organe de collecte.

Dans l'ensemble du texte, y compris les revendications, l'expression « comportant un » doit se comprendre comme étant synonyme de « comportant au moins un », sauf si le contraire est spécifié.

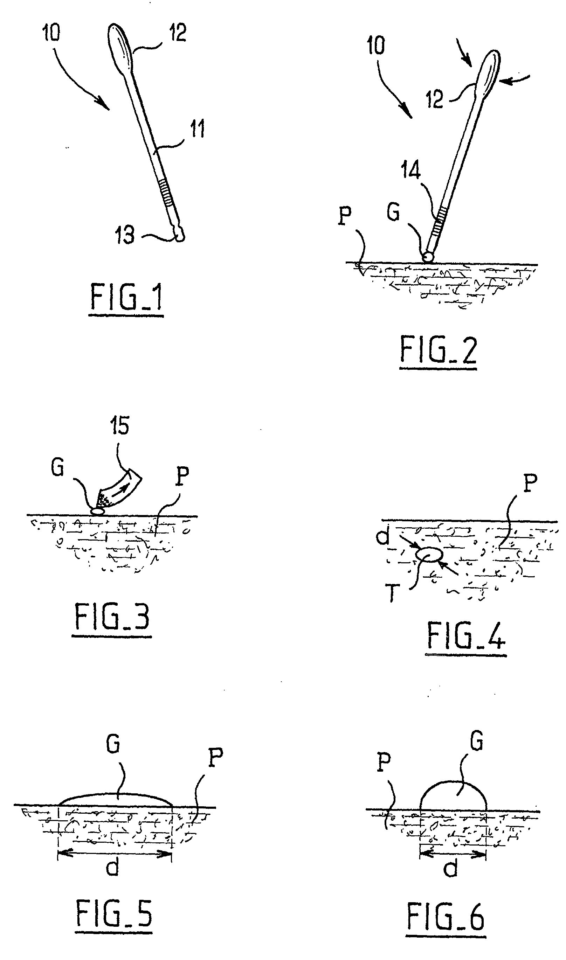

On a représenté sur la figure 1 un exemple d'un dispositif 10 permettant d'appliquer sur la peau une goutte G d'un produit coloré. Ce dispositif 10 se présente sous la forme d'une sorte de pipette comportant un tube 11 communiquant à une extrémité avec un réservoir 12 et pourvu à l'autre extrémité d'une partie sécable 13. La paroi du réservoir 12 est par exemple déformable, de manière à permettre à l'utilisateur de comprimer celui-ci pour faire sortir une goutte de produit, après enlèvement de la partie sécable 13, comme illustré sur la figure 2.

Le tube 11 peut comporter des graduations 14 dont l'utilité sera expliquée plus loin.

Le volume de la goutte G apte à être déposée sur la peau est compris par exemple entre 5 et 100 µl, étant par exemple de l'ordre de 20 µl.

Après avoir déposé sur la peau une goutte G de produit, on laisse cette dernière un temps suffisant pour que, après enlèvement de la goutte G au moyen par exemple d'une bandelette de papier buvard 15, comme illustré sur la figure 3, on puisse observer une tache T due au contact du produit avec la peau. Cette tache T est par exemple sensiblement circulaire et présente un certain diamètre d, comme illustré sur la figure 4.

Lorsque le produit contenu dans le réservoir 12 est un produit aqueux, par exemple une solution d'érithrosine B dans l'eau, l'étendue de la surface de contact entre la goutte G et la peau est différente, selon que celle-ci est sèche ou grasse.

En effet, dans le cas d'une peau grasse, les caractères lipophiles et hydrophiles de la peau peuvent être plus forts. Ainsi, sur la peau du front par exemple, la goutte G tend à s'étaler davantage si la peau est très grasse, comme illustré à la figure 5, tandis que si la peau est peu grasse, la surface de contact est plus faible, comme illustré sur la figure 6.

On comprend ainsi qu'en mesurant, après enlèvement de la goutte G, le diamètre d de la tache T laissée sur la peau, on peut avoir une indication sur le caractère plus ou moins gras de celle-ci.

A titre d'exemple, si l'on dépose sur le dessus de l'épaule une goutte dont le volume est d'environ 20 µl, d'une solution d'érithrosine B dans l'eau à la concentration de 1 mg/ml pendant une durée de 1 mn environ, puis que l'on procède à l'enlèvement de la goutte au moyen d'une bandelette de papier buvard, on peut observer une tache dont le diamètre est de l'ordre de 5 mm environ pour une peau que l'on peut considérer comme "normale", ce diamètre pouvant atteindre par exemple 3 mm environ pour une peau dite "sèche", et pouvant atteindre par exemple 7 mm environ pour une peau dite "grasse".

L'invention n'est pas limitée à la forme de réalisation de l'applicateur représentée aux figures 1 et 2.

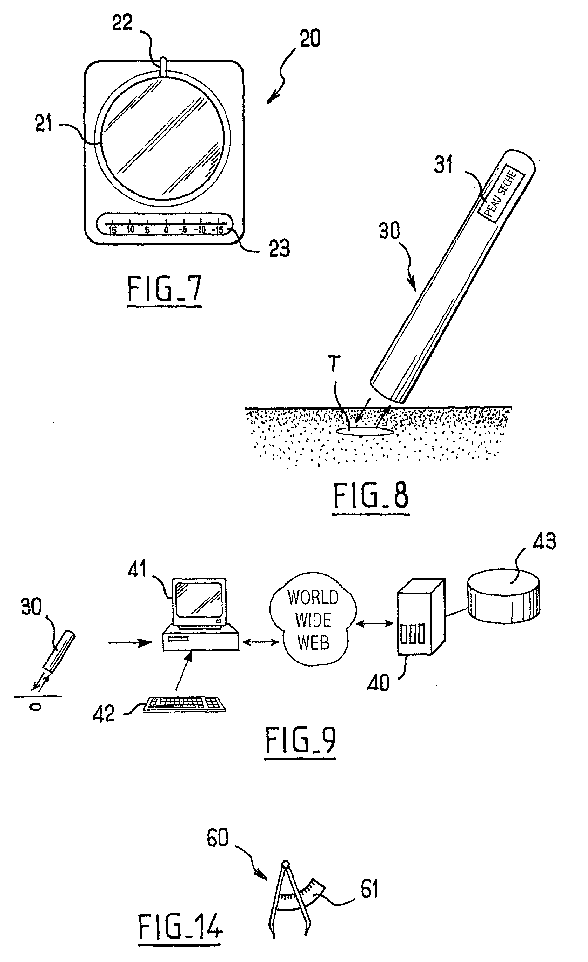

A titre d'exemple, on a représenté une autre forme de réalisation sur la figure 7.

Sur cette figure, on a représenté un applicateur 20 comportant un réservoir 21 formé par exemple entre deux feuilles souples assemblées à leur périphérie, par exemple par thermosoudage.

Le réservoir 21 communique avec un conduit 22 formé entre les deux feuilles. L'extrémité libre de ce conduit peut être découpée, arrachée, percée, ou encore s'ouvrir lorsque l'utilisateur appuie sur le réservoir 21.

L'applicateur 20 peut également servir de support à une échelle 23. Cette échelle 23 peut par exemple être autocollante et être décollée de son support pour être appliquée sur la peau à côté de la tache formée par la goutte de produit déposée sur la peau, pour en évaluer la taille.

L'invention n'est pas limitée à une mesure des dimensions d'une tache laissée par la goutte sur la peau au moyen d'une échelle placée à côté de la tache.

On peut notamment utiliser d'autres moyens pour évaluer les dimensions de la tache, par exemple un appareil de mesure 30 représenté à la figure 8, un tel appareil présentant par exemple la forme générale d'un stylo, étant apte à effectuer par exemple une mesure optique de la tache et pouvant comporter un afficheur 31 permettant de délivrer une information concernant le type de peau.

Une fois la dimension de la tache mesurée, celle-ci peut être affichée telle quelle, en mm, par l'afficheur 31 ou convertie en une information plus élaborée, telle que par exemple un score représentatif du caractère plus ou moins gras de la peau ou une indication non numérique telle que par exemple "peau grasse", "peau normale" ou "peau sèche".

On peut encore utiliser une caméra, par exemple de type « Webcam », pour mesurer la taille de la tache en observant la peau à une distance prédéterminée, éventuellement après avoir placé un élément de dimensions connues à côté de la tache, le nombre de pixels correspondant à la tache étant transformé en une information représentative des dimensions de la tache.

L'appareil de mesure peut délivrer directement une information concernant la taille de la tache ou le type de peau ou transmettre une information, éventuellement sans l'afficher, à un dispositif de traitement et d'affichage distant, la transmission des informations pouvant s'effectuer par fil ou sans fil. On peut notamment prévoir une interface entre un appareil qui effectue une mesure et un dispositif permettant de traiter le résultat des mesures, par exemple un ordinateur personnel ou un serveur distant.

A titre d'exemple, on a représenté sur la figure 9 de manière schématique un ordinateur personnel 41 relié par le réseau Internet à un serveur distant 40. L'ordinateur personnel 41 peut recevoir une information concernant la dimension de la tache laissée sur la peau, cette information étant par exemple entrée au moyen d'un clavier 42 ou transmise par un appareil de mesure tel que par exemple l'appareil 30 représenté à la figure 8.

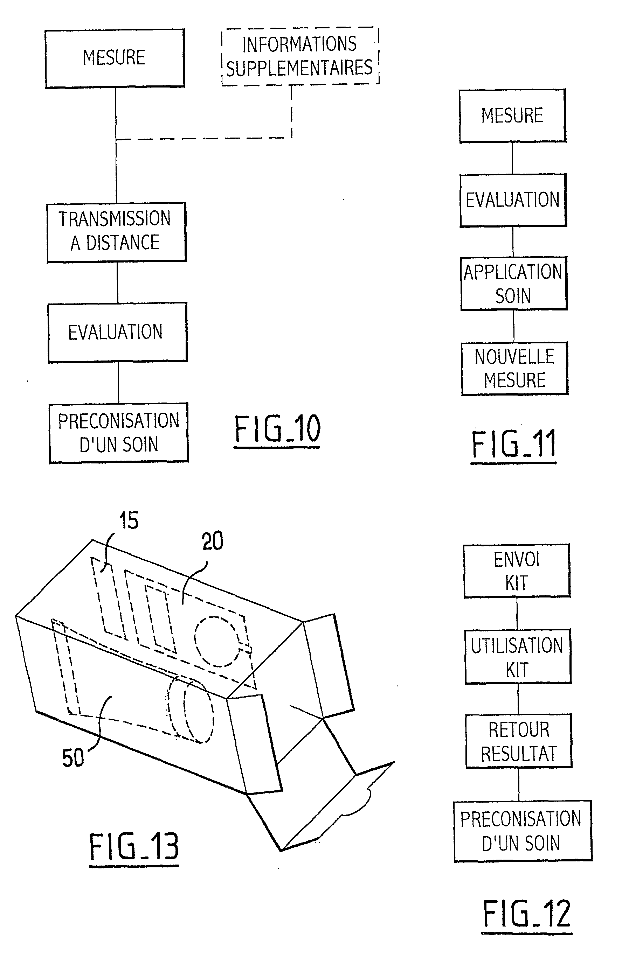

Le serveur distant 40 peut par exemple être programmé pour déterminer en fonction du résultat de la mesure, et en fonction éventuellement d'informations supplémentaires telles que par exemple l'âge, le sexe, la couleur de peau de la personne examinée, le type de peau et préconiser un soin, comme illustré par le schéma en blocs de la figure 10. Les informations supplémentaires peuvent être entrées par exemple en répondant à un questionnaire s'affichant sur l'écran de l'ordinateur 41. L'évaluation des résultats de la mesure, en fonction des éventuelles informations supplémentaires, peut le cas échéant s'effectuer en faisant appel à des informations contenues dans une banque de données 43.

On peut également, moyennant l'emploi d'un produit adéquat, transférer sur un support une empreinte de la trace laissée sur la peau par la goutte de produit, pour effectuer une mesure indirecte de la taille de cette trace.

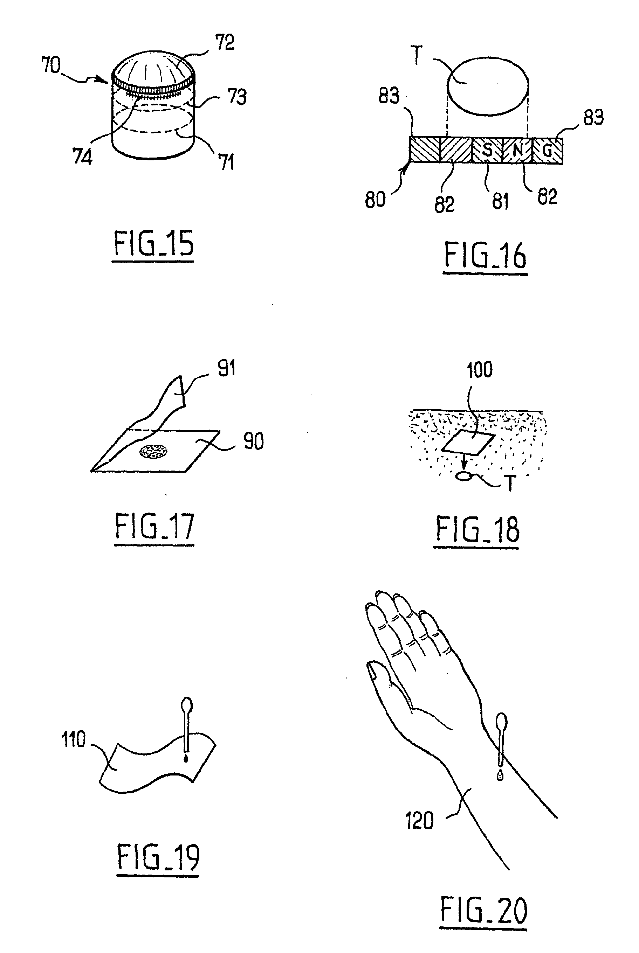

A titre d'exemple, on a représenté à la figure 17 un support 90 présentant par exemple une face adhésive apte à être recouverte par une pellicule de protection amovible 91.

La face adhésive du support peut être appliquée sur la peau après enlèvement de la goutte de produit. La taille de la tache transférée sur le support peut ensuite être mesurée par tout moyen, éventuellement en utilisant des graduations présentes sur le support.

Le cas échéant, la pellicule de protection peut être rabattue sur la face adhésive après transfert de la trace, puis l'ensemble adressé par courrier à un centre de diagnostic.

On peut procéder à des mesures successives de la taille de la tache laissée sur la peau par des gouttes de produit appliquées sur celle-ci à différents instants, afin par exemple de déterminer l'efficacité d'un produit, par exemple un produit de soins, comme illustré sur le schéma en blocs de la figure 11.

On peut notamment procéder à une première mesure des dimensions de la tache afin de déterminer le type de peau, appliquer un soin visant par exemple à diminuer la sécrétion de sébum dans le cas où la peau est estimée trop grasse ou à apporter des lipides dans le cas où la peau est estimée trop sèche, puis effectuer une nouvelle mesure. Si la dimension de la tache, telle qu'elle résulte de cette nouvelle mesure, correspond à une peau dite "normale", le produit qui a été appliqué peut être considéré comme adapté. Si la taille de la tache correspond à une peau trop grasse ou trop sèche, on peut éventuellement modifier le dosage du produit ou changer de produit.

L'évaluation de la peau peut par exemple s'effectuer dans un institut de beauté ou être effectuée par la consommatrice elle-même. On peut par exemple envoyer un kit à la consommatrice, que celle-ci utilise chez elle, et retourner le résultat des mesures à un centre de diagnostic qui va préconiser un soin, comme illustré par le schéma en blocs de la figure 12. Le kit envoyé à la consommatrice peut comporter par exemple un applicateur et des moyens permettant de mesurer la taille de la tache laissée sur la peau après enlèvement ou disparition de la goutte, ainsi éventuellement qu'une bandelette de papier buvard ou tout autre moyen permettant de procéder à l'enlèvement de la goutte. Le kit peut encore comporter un support permettant, par contact avec la peau, d'acquérir une image de la trace laissée par la goutte sur la peau. Un support peut aussi constituer une carte réponse, le cas échéant.

On peut, aussi, comme illustré sur la figure 13, fournir avec un dispositif de conditionnement d'un produit, par exemple un étui en carton ou un blister, un applicateur permettant d'appliquer une goutte de produit coloré sur la peau, des moyens permettant de mesurer les dimensions de la tache laissée sur la peau après enlèvement de la goutte et des moyens permettant d'enlever la goutte, l'applicateur étant par exemple celui représenté à la figure 7 et les moyens permettant d'enlever la goutte étant par exemple constitués par une bandelette de papier buvard 15. L'étui peut encore contenir un support de transfert tel que par exemple celui représenté à la figure 17.

Bien entendu, l'invention n'est pas limitée aux exemples qui viennent d'être donnés.

Le produit appliqué sur la peau peut modifier les propriétés optiques de la peau non pas dans le spectre visible mais dans l'infrarouge ou dans l'ultraviolet, par exemple.

Ainsi, la tache laissée sur la peau après enlèvement de la goutte peut être observable seulement dans l'infrarouge ou l'ultraviolet, ou devenir apparente dans le visible lorsque l'on éclaire avec une lumière infrarouge ou ultraviolette. La tache peut éventuellement être fluorescente, le produit utilisé comportant par exemple du chlorure de Dansyl, de la fluoresceïne ou de la rhodamine.

On peut aussi appliquer sur la peau non pas un produit coloré mais un produit apte à produire une coloration sur la surface de peau exposée à ce produit. Le produit utilisé peut être apte à produire une coloration sur la peau, et comporter par exemple un oxydant capable de s'oxyder à l'air ou un agent oxydant (par exemple PPD et péroxyde d'hydrogène). Un produit réducteur pourrait également être utilisé. On pourrait encore utiliser un substrat d'une enzyme naturellement présente à la surface de la peau, par exemple du glucosidase du glucose, et utiliser un révélateur du glucose libéré. On pourrait encore utiliser un polyphénol et utiliser un polyphénol oxydase pour révéler la taille de la tache.

On peut également utiliser tout autre produit apte à modifier des propriétés physico-chimiques de la peau, par exemple un produit apte à modifier d'autres caractéristiques que la couleur, par exemple le microrelief de la peau ou ses propriétés électriques, capacitives, inductives ou magnétiques.

Pour modifier le microrelief de la peau, on peut utiliser par exemple un acide ou une base. Pour modifier les propriétés magnétiques de la peau, le produit utilisé peut comporter des particules magnétiques, notamment des particules ferromagnétiques.

Le produit peut aussi contenir des anticorps, notamment des anticorps dirigés contre les kératines, sur lesquels on a greffé un ligand ferromagnétique ou chromophore. Cela peut permettre d'améliorer par exemple la fixation du produit sur la peau, en vue d'une révélation ultérieure.

On peut encore utiliser un produit apte à laisser une trace sur la peau dont l'étendue ne devient apparente qu'en exposant cette trace à un révélateur. On peut ainsi utiliser, par exemple, un produit présentant des propriétés adhésives, et utiliser comme révélateur une poudre déposée sur la peau après enlèvement de la goutte. La poudre va adhérer plus fortement à la peau au niveau de la surface qui a été en contact avec la goutte de produit adhésif, ce qui permettra de faire apparaître une tache dont on pourra mesurer les dimensions. L'utilisation d'un produit adhésif peut encore permettre le transfert sur un support d'une trace adhésive, qui pourra être révélée également au moyen d'une poudre, par exemple. L'utilisation d'un produit adhésif peut encore permettre, le cas échéant, en amenant la peau au contact d'un revêtement transférable porté par un support, de faire apparaître la trace du produit par transfert du revêtement sur la peau.

On peut utiliser un produit réalisé avec un solvant autre que de l'eau, par exemple un solvant alcoolique ou un solvant lipidique (par exemple glycérol, iodooctane, iodométhane, octanol), en fonction par exemple de la nature du colorant.

On peut utiliser encore d'autres moyens permettant de mesurer la taille de la tache, par exemple un compte-fils ou un compas.

Un exemple de compas est donné à la figure 14. Sur cette figure, on a représenté un compas 60 comportant une échelle 61 permettant de mesurer l'écartement angulaire des branches. L'échelle 61 peut comporter des indications non numériques et indiquer par exemple directement le type de peau.

L'échelle peut être associée, le cas échéant, à une optique et former un dispositif similaire à un compte-fils.

A titre d'exemple, on a représenté sur la figure 15 un dispositif 70 comportant deux lentilles 71 et 72 et un élément intermédiaire transparent 73 sur lequel est gravée ou imprimée une échelle 74. Cette dernière se trouve par exemple dans le plan focal objet de la lentille 72 et dans le plan focal image de la lentille 71, ce qui permet à l'observateur d'obtenir une image agrandie de la tache et de l'échelle.

Dans les exemples précédents, l'échelle peut comporter une graduation, étant par exemple graduée en millimètres. L'utilisateur peut ensuite transformer la valeur mesurée en une information concernant le type de peau, en utilisant une table de correspondance, par exemple. L'échelle peut aussi comporter des indications permettant de déterminer directement le type de peau.

A titre d'exemple, on a représenté sur 1a figure 16 une échelle 80 qui répond à cette définition. L'échelle 80 comporte plusieurs régions colorées différemment, à savoir une région centrale 81 d'une certaine couleur, dont l'étendue correspond à une peau sèche, deux zones médianes 82 de part et d'autre de cette région centrale, dont l'étendue correspond à une peau normale, et deux régions extrêmes 83 dont l'étendue correspond à une peau grasse. Des lettres ou d'autres inscriptions peuvent être associées aux différentes régions, afin de signaler à quoi elles correspondent, par exemple les lettres « N », « G » et « S ».

Pour utiliser l'échelle 80, l'utilisateur place cette dernière à côté de la tache T, et détermine quelles sont les régions de l'échelle en regard desquelles la tache T s'étend, la région centrale étant positionnée en regard du centre de l'échelle.

Dans l'exemple illustré, la tache T correspond à une peau normale, car la tache T s'étend seulement jusqu'aux régions médianes 82.

On peut encore utiliser un capteur non optique 100, comme illustré à la figure 18.

Le capteur non optique peut comporter par exemple une juxtaposition de cellules de détection élémentaires, de telles cellules pouvant être capacitives, résistives, thermosensibles, ou autres. Des capteurs de ce type sont commercialisés notamment par la société SGS THOMSON MICRO ELECTRONICS sous la marque TOUCH CHIP.

Le capteur non optique 100 peut permettre d'acquérir une image du microrelief de la peau.

Le capteur non optique 100 peut encore, dans une variante, permettre d'acquérir une image d'une trace laissée sur la peau par un produit présentant des propriétés magnétiques.

On peut encore utiliser un capteur non optique intégré à un téléphone portable par exemple, un tel capteur étant utilisé normalement pour identifier le propriétaire du téléphone. Le téléphone peut recevoir un programme permettant d'utiliser le capteur non optique pour déterminer l'étendue de la surface de la peau exposée à une goutte d'un produit choisi pour laisser, après enlèvement, une trace détectable par ledit capteur non optique.

Dans les exemples de mise en oeuvre de l'invention décrits en référence aux figures 1 à 18, une goutte de produit est appliquée sur la zone de peau dont on cherche à déterminer le type, par exemple l'épaule, ou le front ou l'avant-bras. On ne sort pas du cadre de la présente invention lorsque la goutte de produit est appliquée sur un organe de collecte préalablement mis au contact d'une zone de peau, de manière à ce qu'il y ait transfert éventuel du sébum présent sur ladite zone de peau sur l'organe de collecte. La description qui précède, effectuée en référence aux figures 1 à 18, vaut également dans le cas où le produit est appliqué sur l'organe de collecte.

L'organe de collecte peut être constitué par exemple par une feuille 110, comme illustré à la figure 19. Une telle feuille peut être à base de fibres de cellulose, par exemple. Elle peut être passée sur une zone de peau riche en sébum, telle que le front par exemple, puis une goutte de produit peut être déposée sur elle et l'on peut procéder à une mesure de l'étendue de la surface de la feuille au contact du produit, à l'instar de ce qui a été décrit précédemment. Le cas échéant, la feuille peut comporter des graduations ou une échelle prédéterminée.

L'organe de collecte peut également être constitué par une partie du corps humain, par exemple un avant-bras 120, comme illustré sur la figure 20.

L'avant-bras peut être passé sur le front dans un premier temps, afin de se charger du sébum présent sur le front, puis une goutte de produit peut être déposée dessus, afin d'évaluer l'étendue de la surface de peau venant au contact du produit.

Le cas échéant, une goutte de produit peut être déposée sur l'autre avant-bras, et les résultats des évaluations comparés.

## Revendications

1. Procédé permettant de déterminer un type de peau, **caractérisé par le fait qu'**il comporte les étapes suivantes :
- appliquer sur une zone de peau ou sur un organe de collecte préalablement mis au contact de ladite zone au moins une goutte (G) d'un produit apte à modifier au moins une propriété physico-chimique de la surface de ladite zone ou de l'organe de collecte exposée audit produit,
- évaluer, après disparition ou enlèvement de la goutte, l'étendue de ladite surface,
- déterminer en fonction de ladite étendue le type de peau.

2. Procédé selon la revendication 1, **caractérisé par le fait que** ledit produit est un produit coloré ou apte à produire une coloration sur la peau ou sur l'organe de collecte.

3. Procédé selon la revendication 2, **caractérisé par le fait que** le produit est coloré et **par le fait que** la coloration du produit est obtenue par incorporation dans un solvant de l'un des colorants suivants : Erythrosine B, indigo carmine, permanganate de potassium, tanins, notamment les produits de l'oxydation des polyphénols, mélanines, henné, anthocyanes, produits fluorescents, notamment le chlorure de Dansyl.

4. Procédé selon l'une quelconque des revendications précédentes, **caractérisé par le fait que** le produit présente des propriétés magnétiques.

5. Procédé selon la revendication 4, **caractérisé par le fait que** le produit comporte des particules magnétiques, notamment des particules contenant du fer.

6. Procédé selon l'une quelconque des revendications précédentes, **caractérisé par le fait que** le produit comporte au moins un anticorps, notamment un anticorps spécifique des kératines.

7. Procédé selon la revendication 6, **caractérisé par le fait que** ledit anticorps est associé à l'un des éléments de la liste suivante : particules magnétiques, composé fluorescents, enzymes, système de type "ELISA".

8. Procédé selon l'une quelconque des revendications précédentes, **caractérisé par le fait que** le produit utilisé est apte à modifier le microrelief de ladite zone de peau ou de l'organe de collecte.

9. Procédé selon l'une quelconque des revendications 1 à 8, **caractérisé par le fait que** le volume de la goutte de produit déposée sur la peau ou sur l'organe de collecte est compris entre 5 et 100 µl, de préférence entre 10 et 60 µl.

10. Procédé selon l'une quelconque des revendications précédentes, **caractérisé par le fait que** la goutte (G) est enlevée au moyen d'un élément absorbant (15).

11. Procédé selon l'une quelconque des revendications précédentes, **caractérisé par le fait que** le produit utilisé est coloré ou apte à produire une coloration sur la peau ou l'organe de collecte et **par le fait que** l'on mesure la taille de la tache laissée par la goutte à l'aide d'un compas, d'une caméra, d'un rayon lumineux ou d'une échelle, graduée ou non, éventuellement associée à une optique.

12. Procédé selon l'une quelconque des revendications précédentes, **caractérisé par le fait que** le résultat de la mesure est transmis à un ordinateur à des fins d'analyse.

13. Procédé selon l'une quelconque des revendications précédentes, **caractérisé par le fait que** le résultat de la mesure est transmis à distance, par courrier ou par réseau informatique, notamment sur le réseau Internet.

14. Procédé selon l'une quelconque des revendications précédentes, **caractérisé par le fait qu'**il comporte l'étape de préconisation d'un soin.

15. Procédé selon l'une quelconque des revendications précédentes, **caractérisé par le fait que** ladite étendue est évaluée in situ.

16. Procédé selon l'une quelconque des revendications 1 à 14, **caractérisé par le fait que** ladite étendue est évaluée après transfert sur un support d'une trace laissée sur ladite zone de peau ou l'organe de collecte (110 ; 120) par ladite goutte de produit.

17. Procédé selon l'une quelconque des revendications précédentes, **caractérisé par le fait que** la goutte de produit est appliquée directement sur ladite zone de peau.

18. Procédé selon l'une quelconque des revendications 1 à 16, **caractérisé par le fait que** la goutte de produit est appliquée sur un organe de collecte (110 ; 120) préalablement mis en contact avec ladite zone de peau.

19. Procédé selon la revendication 18, **caractérisé par le fait que** l'organe de collecte est constitué par une partie du corps humain, notamment un avant-bras.

20. Procédé selon la revendication 19, **caractérisé par le fait que** l'on effectue des évaluations comparatives, en comparant les résultats d'une évaluation sur l'organe de collecte avant le transfert de sébum de ladite zone de peau sur l'organe de collecte et d'une évaluation après transfert.

21. Procédé selon la revendication 19, **caractérisé par le fait que** l'on compare les résultats d'une évaluation sur un avant-bras (120) après passage sur le front et d'une évaluation sur l'autre avant-bras.

22. Procédé selon la revendication 18, **caractérisé par le fait que** l'organe de collecte est constitué par un support (110) indépendant du corps humain, notamment une feuille.

23. Procédé de traitement cosmétique de la peau, **caractérisé par le fait qu'**il comporte les étapes suivantes :
- appliquer sur une zone de peau ou sur un organe de collecte (110 ; 120) préalablement mis au contact avec ladite zone au moins une goutte d'un produit de test apte à modifier au moins une propriété physico-chimique de la surface de ladite zone de peau ou de l'organe de collecte exposée audit produit de test, notamment un produit coloré ou apte à produire une coloration sur ladite zone de peau ou l'organe de collecte,
- évaluer après disparition ou enlèvement de la goutte, l'étendue de la surface de ladite zone de peau ou de l'organe de collecte ayant été au contact de ladite goutte,
- déterminer en fonction de ladite étendue le type de peau,
- traiter la peau avec un produit cosmétique en fonction du type de peau ainsi déterminé.

24. Procédé selon la revendication précédente, **caractérisé par le fait que** la goutte de produit de test est appliquée sur l'avant-bras dans le cas où le produit de traitement est destiné à l'ensemble du corps.

25. Procédé selon la revendication 23, **caractérisé par le fait que** la goutte de produit de test est appliquée sur le front dans le cas où le produit de traitement est destiné plus particulièrement au visage.

26. Procédé pour déterminer l'efficacité d'un traitement, notamment un traitement apte à agir sur l'hydratation et/ou la teneur en lipides de la peau, **caractérisé par le fait qu'**il comporte les étapes suivantes :
- appliquer sur une zone de peau ou sur un organe de collecte préalablement mis en contact avec ladite zone au moins une goutte d'un produit apte à modifier au moins une propriété physico-chimique de la surface de ladite zone de peau ou de l'organe de collecte au contact de ladite goutte,
- évaluer l'étendue de la surface de ladite zone de peau ou de l'organe de collecte ayant été au contact de ladite goutte après enlèvement ou disparition de celle-ci, par observation des modifications induites par ledit produit,
- effectuer le traitement,
- renouveler les étapes a) et b) sur la peau traitée,
- déduire de la comparaison des résultats avant et après traitement une indication sur l'efficacité du traitement.

27. Procédé pour évaluer l'âge physiologique de la peau, **caractérisé par le fait qu'**il comporte les étapes suivantes :
- appliquer sur une zone de peau ou sur un organe de collecte préalablement mis en contact avec ladite zone au moins une goutte d'un produit apte à modifier au moins une propriété physico-chimique de la surface de ladite zone de peau ou de l'organe de collecte exposée audit produit,
- évaluer, après disparition ou enlèvement de la goutte, par observation des modifications induites par ledit produit, l'étendue de la surface de ladite zone de peau ou de l'organe de collecte ayant été au contact de ladite goutte,
- déterminer en fonction de ladite étendue l'âge de la peau.

28. Ensemble comportant un applicateur apte à appliquer sur la peau ou un organe de collecte une goutte d'un produit apte à modifier au moins une propriété physico-chimique de la surface de la peau ou de l'organe de collecte exposée audit produit, notamment un produit coloré ou apte à produire une coloration sur la peau ou l'organe de collecte, et au moins une échelle ou un appareil de mesure pour évaluer l'étendue de la surface de la peau ou de l'organe de collecte ayant été au contact de ladite goutte (G), après disparition ou enlèvement de ladite goutte.

29. Ensemble selon la revendication 28, **caractérisé par le fait que** l'applicateur comporte un réservoir (12 ; 21) de produit et une partie sécable ou destinée à être coupée ou percée.

30. Ensemble selon la revendication 28 ou 29, **caractérisé par le fait que** ladite échelle est portée par l'applicateur.

31. Ensemble selon l'une quelconque des revendications 28 à 30, **caractérisé par le fait que** ladite échelle est autocollante.

32. Ensemble selon l'une quelconque des revendications 28 à 31, **caractérisé par le fait que** ladite échelle (80) comporte un marquage apte à indiquer directement le type de peau.

33. Ensemble selon l'une quelconque des revendications 28 à 32, **caractérisé par le fait que** l'applicateur comporte un réservoir de produit formé au contact d'au moins une paroi souple.

34. Ensemble selon la revendication 33, **caractérisé par le fait que** ledit réservoir de produit est formé entre deux feuilles superposées.

35. Ensemble selon l'une quelconque des revendications 28 à 34, **caractérisé par le fait que** l'applicateur comporte un conduit par lequel sort la goutte, ayant un diamètre intérieur compris entre 50 µm et 3 mm, de préférence entre environ 0,1 mm et environ 2 mm.

36. Ensemble selon l'une quelconque des revendications 28 à 35, **caractérisé par le fait que** l'appareil de mesure comporte une caméra.

37. Ensemble selon l'une quelconque des revendications 28 à 36, **caractérisé par le fait que** l'appareil de mesure comporte au moins un capteur magnétique.

38. Ensemble selon l'une quelconque des revendications 28 à 37, **caractérisé par le fait que** l'appareil de mesure comporte au moins un capteur non optique, apte à délivrer une image des microreliefs de la peau.

39. Ensemble selon l'une quelconque des revendications 28 à 38, **caractérisé par le fait que** l'appareil de mesure comporte en outre une interface permettant de transmettre des données à un ordinateur personnel et/ou à un réseau informatique.

40. Kit comportant un ensemble tel que défini dans l'une quelconque des revendications 28 à 39 et un produit à appliquer sur la peau, notamment un produit de soins.

41. Appareil de mesure de l'étendue d'une surface de peau ou d'un organe de collecte ayant au moins une propriété physico-chimique modifiée par rapport à une zone de la peau ou de l'organe de collecte située autour de ladite surface, notamment un appareil de mesure de la taille d'une tache colorée sur la peau ou sur l'organe de collecte, cet appareil comportant des moyens de mesure de l'étendue de ladite surface et des moyens d'affichage d'un type de peau en fonction du résultat de la mesure.

42. Appareil selon la revendication 41, **caractérisé par le fait qu'**il est agencé pour délivrer une information non numérique, par exemple du type « peau sèche », « peau normale » ou « peau grasse ».

43. Echelle comportant un marquage propre à indiquer directement le type de peau.

44. Echelle selon la revendication 43, **caractérisée par le fait qu'**elle est associée à une optique (71, 72) permettant de visualiser simultanément une image agrandie de la tache et l'échelle.

45. Compas (60), **caractérisé par le fait qu'**il comporte des indications propres à indiquer directement le type de peau.
